Europäisches Patentamt

European Patent Office

Office européen des brevets

(19)

(11) Publication number: **0 270 326**
**A2**

(12) **EUROPEAN PATENT APPLICATION**

(21) Application number: 87310533.2

(22) Date of filing: 30.11.87

(51) Int. Cl.⁴: **B65D 25/10**

(30) Priority: 05.12.86 US 938187

(43) Date of publication of application:
08.06.88 Bulletin 88/23

(84) Designated Contracting States:
BE CH DE FR GB IT LI LU NL SE

(71) Applicant: **Nalge Company**
**75 Panorama Creek Drive P O Box 20365**
**Rochester New York 14602(US)**

(72) Inventor: **Leoncavallo, Richard Anthony**
**45 Monroe Avenue**
**Pittsford New York 14534(US)**
Inventor: **Kover, Linda Jean**
**322 Ravenwood Avenue**
**Rochester New York 14619(US)**
Inventor: **Phillips, Gregory Robert**
**2080 Penfield Road**
**Penfield New York 14526(US)**

(74) Representative: **Oliver, Roy Edward et al**
**POLLAK MERCER & TENCH High Holborn**
**House 52-54 High Holborn**
**London WC1V 6RY(GB)**

(54) **Storage container.**

㉗ A plastics material container (10), for holding an array of vials or other like articles, has a base portion (14) and a top cover (12), the base portion being provided with a divider (38) defining a plurality of openings (40) for receiving and maintaining in position a plurality of vials or other like articles; the divider is provided with indicia (43) for identifying the positions of the vials.

FIG. I

## STORAGE CONTAINER

This invention relates to storage containers for holding a plurality of vials or other like articles having an inventory system which allows quick and easy removal of the vials stored therein.

### BACKGROUND OF THE INVENTION

In the medical and biological research fields, it is often necessary to store and preserve numerous culture samples or biological specimens for later evaluation, inspection and/or use. Generally these specimens and/or samples are placed in individual glass or plastics vials or other like containers. A plurality of these vials are them placed into a single container which is placed in a mechanical freezer or liquid nitrogen. In the past, these containers have been made of cardboard or stainless steel. As part of the normal investigation work of a researcher, it is often necessary to retrieve one or several particular vials out of the group of vials in any particular storage container. An inventory system of the prior art generally takes the form of a written list identifying the contents of each vial in a particular container and some type of identifying position associated therewith. Generally the vials placed in the containers are arranged in some sort of an array, for example, a 9X9 array of openings for receiving an equal number of vials. Some containers have provisions for providing a label on the top cover for later providing identification of the contents. However, once the cover is removed, there are no other indicia within the container for identifying the position of each individual vial. Further, since these containers are typically square, it is possible to place the cover on so that the label does not correspond to the position of the contents therein. Therefore, in removing a single vial from an array, the user must be very careful to retrieve the particular vial or vials desired. Likewise when inserting vials into the container, care must be taken so that the vial being inserted is in the appropriate position so desired.

With respect to specimens that are cold stored, especially with respect to specimens stored at cryogenic temperatures (approximately -70° Centigrade or lower), it is important that the vials that are not removed from the container be returned to the cryogenic environment as soon as possible. Typically, a cryogenic storage container may have up to 81 different vials. Therefore, it is possible that a particular vial will be removed and placed back into the cryogenic environement many times prior to its actual removal and use. In order to assure the viability of the specimen being stored, the time period in which the specimens are out of the cryogenic environment should be minimized. Typically these specimens are sensitive to temperature change. If a particular sample is taken out numerous times and allowed to stay out for a particularly long time, the viability of the specimens will deteriorate substantially, possibly to the point where recovery is no longer possible. Accordingly, it is important for the vials that are not to be used to be removed from the cryogenic environment, preferably, for no more than approximately 30 seconds. Additionally, the storing of containers in a cold environment greatly inhibits, if not prevents, the writing and/or applying of a label thereon.

Applicants have invented a container having an improved inventory system which minimizes the problem of the prior art and is particularly adapted for use as a cryogenic storage container.

### SUMMARY OF THE INVENTION

A plastic container for holding an array of vials or other like articles. The container has a base portion and a top cover for placement thereon. The base portion is provided a divider defining a plurality of openings for receiving and maintaining in position a plurality of vials or other like articles. A divider is provided with indicia for identifying the position of the vials.

### DESCRIPTION OF THE DRAWINGS

Figure 1 is a top plan view of the container of the present invention;

Figure 2 is a front elevational view of the invention illustrated in figure 1 partially broken away along line 2-2 with the cover in the open position;

Figure 3 is a top view of a base portion of a container of figure 2 taken along line 3-3; and

Figure 4 is a top plan view of a modified form of a container made in accordance with the present invention.

### DETAILED DESCRIPTION OF THE INVENTION

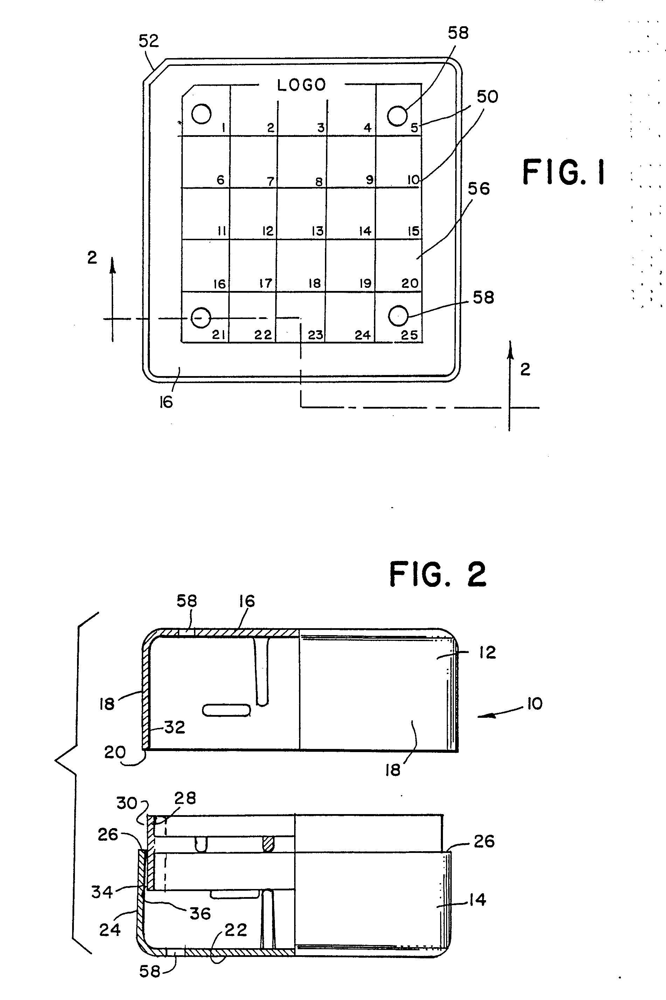

Referrring to figures 1 and 2, there is illustrated a container 10 made in accordance with the present invention. A container 10 is made of a plastics material which is capable of withstanding low cryogenic temperatures and which is also capable of being exposed to temperatures experienced during dishwashing, autoclaving and/or sterilization. In the particular embodiment illustrated, the container 10 is made of polycarbonate. The container 10 comprises of top cover 12 which sits on base portion 14. The cover 12 comprises a top wall 16 and a peripheral outer wall 18 which extends downwardly from top wall 16 and terminates at its lower end in rim portion 20.

The base portion 14 comprises a bottom wall 22 and an upstanding peripheral wall 24 extending therefrom which terminates in a ridge portion 26. When the top cover 12 is placed on base portion 14 in the closed position, the rim 20 of top cover 12 mates with ridge portion 26 of base portion 14. Base portion 14 is further provided with retaining wall 28 which extends about the periphery of wall 24 and extends above ridge portion 26. The outer surface 30 of retaining wall 28 mates with the inside surface 32 of wall 18 when top cover 12 is placed on base portion 14 in the closed position. The retaining wall 28 is constructed so as to snap fit into the wall 24 of base portion 14. The outer surface 30 of retaining wall 28 at its lower ends is provided with a plurality of projection 34 (see figure 2) which snap into a mating recess 36 formed in wall 24. Retaining wall 28 positively positions the top cover 14 on base portion 14 and prevents axial movement therebetween. In the preferred form of the particular invention illustrated, the retaining wall 20 is a separate piece; however, the retaining wall 28 may be integrally formed with or somehow otherwise secured to the outer peripheral wall 24 of base portion 14.

Integrally formed with retaining wall 28 is divider 38 which is spaced from bottom wall 22 of base portion 14. The divider 38 has a plurality of openings 40 for holding and maintaining in position a plurality of vials or other like articles. In the particular embodiment illustrated, openings 40 are arranged in a 5X5 array where a total 25 separate individual openings 40 are provided. However any desired array may be selected with any desired number of openings. In the particular embodiment illustrated, openings 40 are formed by a plurality of horizontal branches 42 and vertical branches 44 which connect oppositely-disposed sides of retaining wall 28.

Associated with each individual opening 40 is a different indicium 43 for identifying each position. The indicia are permanently fixed to divider 38 so as to prevent accidental removal thereof and disposed directly adjacent each opening. Preferably indicia 43 are placed in the same positions for each opening 40. For example, in the embodiment illustrated, the indicium 43 is disposed at the lower right-hand corner of each opening. However, it is sometimes necessary to place the indica 43 in a different locality, as illustrated in the lower right-hand corner for position 25. In this particular instance due to molding techniques, it is preferable to have the numeral 25 in the upper left-hand corner. In the particular embodiment illustrated, the indicia associated with each opening are numerals. Since the particular embodiment illustrated is a 5X5 array of openings 40, the indica take the form of numerals from 1 through 25. The numerals 1 through 25 are preferably integrally formed in divider 28 which project above the surface of branches 42, 44 as illustrated. However, other permanent marking may be provided, for example, it may be desirable to print indicia 43 with an appropriate permanent ink.

In the particular embodiment illustrated, the numerals are placed at the junction of the branches 42 and 44 where a marking surface 48 is provided for receiving the numerals. The openings 40 identified by numerals 5 and 10 illustrate in dash lines the relative position of a vial placed therein. As can be seen, marking surface 48 does not interfere with the placement of a vial or other like article. It is, of course, understood that the marking surface 48 and branches 42, 44 may be any size desired, so as to accomodate numerous size openings or indicia. One of the primary interests is that these numerals should not interfere with the insertion or removal of vials placed in the openings 40.

Referring to figure 1, it can be seen that the outer surface of top wall 16 is provided with identifying indicia 50 which are identical in identification to the indicia 43 provided on the divider 38. The indicia 50, in the particular embodiment illustrated, are numerals 1 through 25, which are positioned to correspond with the positions of the indica 43 on a divider 38 underneath. As also can be seen, there are provided additional indicia 56 on the cover in the form of a grid system, thereby providing individual areas wherein additional writings may be placed by permanent marking by a writing instrument if so desired. As also illustrated, a space may be provided on top cover 12 identifying marking in the top portion of the cover such as a company logo.

Top cover 12 and bottom cover 14 are further provided with openings 58 which assist in the egress and ingress of a fluid or other, gas medium into the container. For example when the containers are placed in a liquid nitrogen environment, the openings 58 assist in allowing liquid nitrogen to flow within the container and upon removal assist in allowing drainage. In the particular embodiment illustrated, the openings 58 in top cover 12 are disposed directly above the openings 58 in base portion 14.

The providing of numerals on the outside surface of the top cover 12 assists in orienting the box appropriately and to identifying the position of the vials inside. Once the top cover 12 is removed, the divider having indicia 43 thereon further assists in locating and identifying the appropriate positioning of the desired vial or vials.

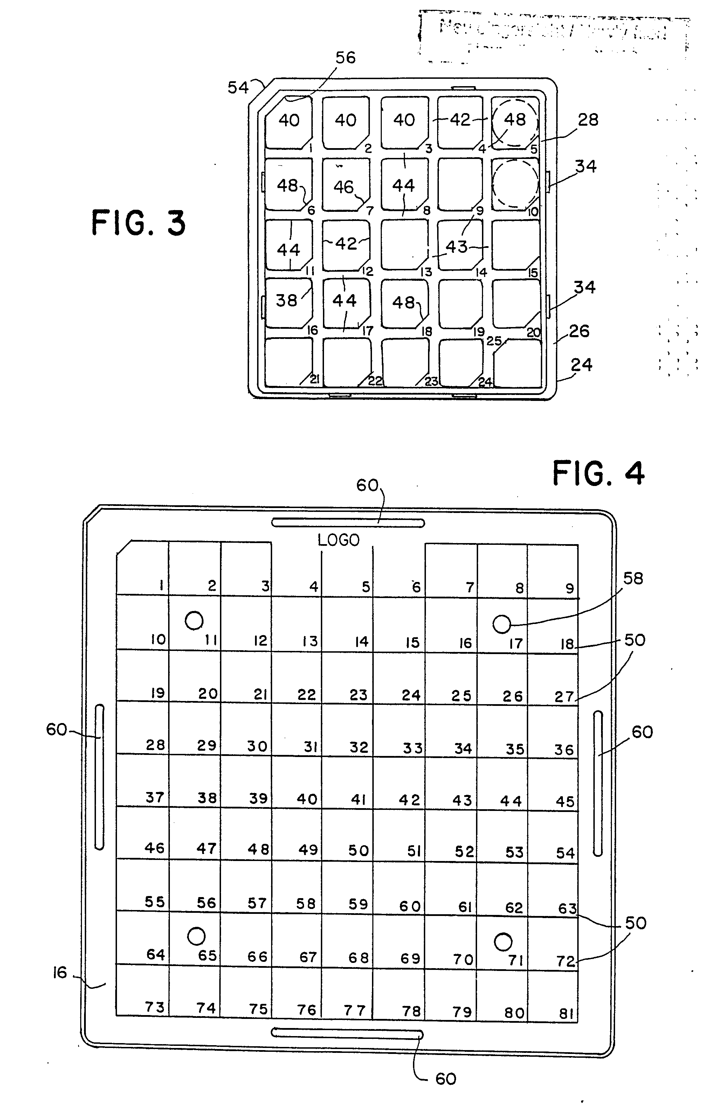

In the particular embodiment illustrated, the overall outer configuration of the container 10 is substantially square, having an outer dimension of about 7.7 cm X 7.7 cm. However, the container may be other shapes, for example, rectangular or hexagonal, or be sized to contain more vials as illustrated in figure 4.

In order to assure that the indicia on top cover 12 directly correspond to the indicia on divider 38, the container 10 is provided with means for permitting closure of the top cover on base portion in only one way. In the particular embodiment illustrated, means for assuring proper orientation of the top cover with respect to the divider is provided by beveling one corner of the base portion 14 and top cover 12. The corner 52 of top cover 12 (see figure 1), is beveled so as to mate with the corner 54 of base portion 14 (see figure 3). Likewise the retaining wall 28 is beveled at corner 26 so as to conform to the configuration of the top and bottom cover. The beveling of one corner provides a simple yet positive manner in which to assure the proper orientation and alignment of the top cover 12 with respect to the base portion 14, so that the indicia on top cover 12 will correspond directly to the indicia on divider 38 directly below. The beveled corner further assists the user in orienting the container so that the container properly faces the user, thus allowing the indica on divider 38 to be easily read. While the preferred embodiment of the present invention illustrates indicia on top cover 12, it may be possible to omit these indicia in favour of an orientation indexing means which is associated with the indica on divider 38. For example, the beveled corners 52, 54 are adjacent the position identified by numeral 1. Therefore, the user will simply orient the container so that the beveled corners 52, 54 are in the appropriate position, so that the indicia on divider 38 can be quickly and easily read when the cover is removed. It is understood that the present invention is not limited to the particular means for controlling the alignment of the cover with the base portion and the orientation of the container. The assuring of the orientation of the indicia on top cover 12 with respect to the indicia on the divider 38 and base portion 14 or orientation of the indicia on the divider when the cover is taken off may be accomplished in any other desired manner.

Referring to figure 4, there is illustrated a modified container 10 made in accordance with the present invention similar to that illustrated in figure 1. Like numerals referring to identical elements. As previously noted, the present invention is particularly adpated for use as a cryogenic storage container. For the reasons previously discussed, it is important that identification and location of the vials within the containers be done as quickly and efficiently as possible. Applicants have unexpectedly found that in larger sizes as illustrated in figure 4, in order to assure quick removal of the top cover from the base portion 14 after it has been removed from the cryogenic environment, it is beneficial to provide a narrow slot 60 on the top cover so as to minimize or prevent warping of the top cover. When the container is first removed from its cryogenic environment, i.e., temperatures below - 70° Centigrade, the top cover has a tendancy to warp and distort. Once the container warms up the warping or distortion subsides. However, precious time has passed in which to obtain the desired vial therein. Applicants have quite unexpectedly found that by the placement of the relatively narrow slots 60, which extend through the cover approximately in the centre of each side around the periphery thereof, this minimizes or eliminates this initial distortion. Accordingly in order to assure quick and easy removal of the cover 14, slots 60 are each approximately 3.81 cm long and have a width of about .15 cm for a container having an overall size of about 13.3 X 13.3 cm. It is believed that the placement of the slots relieves stress experienced by the top cover, when it is initially removed from the cryogenic environment. The particular size, shape and location may vary, however; these slots 60 are designed to relieve stress so as to prevent initial warping upon removal from a cold environment.

Various modifications to the present invention may be made without departing from the scope of the present invention. For example, various other indicia may be used in place of the numerals for identifying the positions of the openings 40. The retaining wall may be designed to be secured to the top cover and mate with the inside surface of the base portion. Additionally, the divider may be secured directly to the base portion. Further, as previously discussed, the particular number of openings or size of the container may be varied as desired.

## Claims

1. A container of plastics material, for holding a plurality of vials or other like articles, characterised by comprising:

(a) a base portion (14) having a bottom wall (22) and an upstanding peripheral outer wall (24) extending upwards from the bottom wall and terminating in a ridge portion (26), an inner retaining wall (28) disposed inwardly of the peripheral wall, a divider (38) disposed within the base portion and having therein a plurality of openings (40) arranged in an array, each of the openings being sized so as to receive a single vial, and a different indicium (43) associated with each opening and permanently affixed to the divider, for identifying each part of the openings and

(b) a top cover (12) for mating with the base portion, having a top wall (16) and a downwardly-extending peripheral wall (18) which terminates in a rim portion (20) for mating with the ridge portion of the base portion, the inside surface of the top cover mating with the retaining wall when the top cover is placed on the bottom portion.

2. A plastics container according to claim 1, having means (52,54) for positioning the top cover on the base portion in only one way, comprising a bevelled corner (52) on the top portion and a bevelled corner (54) on the base portion.

3. A plastics container according to claim 1 or 2, having at least one opening (58), for allowing egress and ingress of a fluid or gas medium therethrough, in each of the bottom wall of the base portion and the top wall of the top cover.

4. A plastics container according to claim 1, 2 or 3, wherein the divider is spaced from the bottom wall within the peripheral wall of the base portion and divides the bottom portion into the plurality of openings, the indicium associated with each opening being provided on the upper surface of the divider.

5. A plastics container according to any of claims 1 to 4, wherein the divider is a separate piece from the base portion and snaps into position in the base portion.

6. A plastics container according to any preceding claim, wherein the outer surface of the top wall of the top cover carries second indicia (50) corresponding to the different first indicia identifying the respective openings.

7. A plastics container according to any preceding claim, wherein the top cover comprises a material capable of receiving and retaining a marking by a writing instrument.

8. A plastics container according to any preceding claim, wherein the retaining wall is designed to mate with the inside surface of the top cover, to prevent axial movement of the top cover when placed on the base portion.

9. A plastics container according to any preceding claim, wherein the top cover is provided with at least one slot (60) along its periphery, for minimizing warping when the top cover is first removed from a cryogenic environment.

10. A plastics container according to any preceding claim, having means for orientating it so that the indicia on the divider are properly oriented for the user.

# FIG. 1

LOGO

| 1 | 2 | 3 | 4 | 5 |
| 6 | 7 | 8 | 9 | 10 |
| 11 | 12 | 13 | 14 | 15 |
| 16 | 17 | 18 | 19 | 20 |
| 21 | 22 | 23 | 24 | 25 |

52
58
50
56
2
16
58
2

# FIG. 2

58  16  12
18  32  10
20  18

30  28
26  26
34  14
24  36  22
58

**FIG. 3**

**FIG. 4**

LOGO